# EUROPÄISCHE PATENTSCHRIFT

(11) **EP 0 510 369 B1**
(45) Veröffentlichungstag und Bekanntmachung des Hinweises auf die Patenterteilung: **19.07.1995**
(21) Anmeldenummer: 92105110.8
(22) Anmeldetag: 25.03.1992
(51) Int. Cl.: F16L 33/20, F16L 37/12, F16L 35/00, A61M 16/08, A61M 5/00

(54) **Kupplungseinrichtung für ein Schlauchsystem**
Coupling device for a flexible tube system
Dispositif d'accouplement pour un système de tuyau flexible

(30) Priorität: 26.04.1991 DE 4113707
(43) Veröffentlichungstag der Anmeldung: 28.10.1992
(73) Patentinhaber: Drägerwerk Aktiengesellschaft, 23542 Lübeck (DE)
(72) Erfinder: Kollenbrandt, Norbert, W-2419 Berkenthin (DE); Schwenke, Heiko, W-2400 Lübeck (DE); Wimmers, Elmar, W-2408 Hemmelsdorf (DE)

(56) Entgegenhaltungen:
- AT-B- 336 359
- DE-A- 3 623 367
- FR-A- 2 605 383
- GB-A- 1 109 105

## Beschreibung

Die Erfindung betrifft eine Kupplungseinrichtung zwischen zwei Teilen eines Schlauchsystems, von denen das erste Teil ein Rohrstutzen mit einer Hinterschneidung und das zweite Teil ein auf den Rohrstutzen geschobener Schlauch mit einem in die Hinterschneidung eingreifenden Rastglied ist.

Eine Kupplungseinrichtung der genannten Art ist aus der DE 36 23 367 C2 bekanntgeworden. Die bekannte Kupplungseinrichtung dient zum Anschluß eines Schlauches an den Rohrstutzen eines Narkose- oder Beatmungsgerätes. Der Schlauch weist an seiner Innenseite ein Rastglied auf, das als elastische Lippe ausgebildet ist, und beim Aufschieben auf den Rohrstutzen in einer Hinterschneidung einrastet. Die Kupplungseinrichtung ist so ausgelegt, daß zwar während der Beatmung der Schlauch fest auf dem Rohrstutzen anliegt, andererseits aber die Möglichkeit besteht, im Notfall die Schläuche schnell vom Rohrstutzen abzuziehen. Hierbei wird durch die axiale Zugkraft an dem Schlauch das Rastglied soweit radial aufgeweitet, bis es aus der Hinterschneidung herausgleitet und damit die Kupplungseinrichtung gelöst wird.

Kupplungseinrichtungen sind auch aus der Verbindung von Atemschläuchen in Atemschutzgeräten bekannt. Hier besteht die Forderung, daß die Atemschläuche im Betrieb fest mit dem zugehörigen Rohrstutzen verbunden sein müssen, bei der Geräteaufbereitung aber ein schnelles Lösen gewünscht wird. Hierzu wird der Schlauch auf dem zugehörigen Rohrstutzen festgeschellt, was einen hohen Montageaufwand bedeutet. Durch zu fest angezogene Schellen kann aber der Schlauch beschädigt werden, und es ist oftmals schwierig, mit Werkzeug in den kompakt aufgebauten Atemschutzgeräten zu hantieren.

Eine in der DE 26 57 215 C3 beschriebene schnell lösbare Kupplung besteht aus einem Rohrstutzen, der mit einer umlaufenden Kerbe versehen ist und einem Schlauch mit einer Kupplungsmuffe, an deren Ende gegenüberliegende Vorsprünge angebracht sind, die im zusammengesetzten Zustand in die Kerbe des Rohrstutzens einrasten. Die Kupplungsmuffe ist im Bereich der Vorsprünge aus elastischem Material gefertigt und mit Druckaufnahmebereichen versehen, die zu den Vorsprüngen um 90 Grad versetzt angeordnet sind. Beim Zusammendrücken der Druckaufnahmebereiche wird die Kupplungsmuffe derart aufgeweitet, daß die Vorsprünge aus der Kerbe herausgleiten.

Die bekannte Kupplung gestattet zwar ein schnelles Zusammenfügen von Schlauch und Rohrstutzen, jedoch können von der Verbindung nur begrenzte Abzugskräfte aufgenommen werden. Bei höheren Abzugskräften weitet sich der äußere, nachgiebige Teil der Kupplungsmuffe radial auf und die Vorsprünge gleiten aus der Kerbe. Außerdem ist eine derartige Kupplung teuer in der Herstellung, da komplizierte, korrespondierend zueinander geformte Kunststoffteile notwendig sind, die in engen Toleranzen gefertigt sein müssen.

Aus der AT-B 336 359 ist eine lösbare Kupplungseinrichtung zwischen zwei Teilen eines Schlauchsystems bekanntgeworden, von denen ein Teil ein spannzangenartiger Rohrstutzen und das andere Teil ein Schlauch ist, der in den Rohrstutzen gesteckt wird und mit einem auf den Rohrstutzen aufschiebbaren Sicherungsring innerhalb des Rohrstutzens festgeklemmt wird. Der aus elastischem Material bestehende Sicherungsring besitzt eine Nut, welche in der Endstellung des Sicherungsringes in eine an der Außenseite des Rohrstutzens befindliche Nase einrastet.

Nachteilig bei der bekannten Kupplungseinrichtung ist, daß der Sicherungsring beim Auswechseln des Schlauches, aufgrund der Spannzangenkonzentration des Rohrstutzens, aus der unter Vorspannung stehenden Nase entfernt werden muß.

Der Erfindung liegt die Aufgabe zugrunde, eine Kupplungseinrichtung derart zu verbessern, daß bei einfacher Lösbarkeit von Rohrstutzen und Schlauch die Abzugskraft äquivalent einer festen Einschellung des Schlauches auf dem Rohrstutzen ist.

Die Lösung der Aufgabe erfolgt dadurch, daß eine hülsenförmige Kupplungsmuffe vorgesehen ist, die den Schlauch im Bereich des Rastgliedes als Sicherungsring schellenförmig umgreift und zur Fixierung auf dem Rohrstutzen an der Innenseite eine umlaufende Kerbe aufweist, daß am Rohrstutzen zwei gegenüberliegend angeordnete Vorsprünge vorgesehen sind, welche in die Kerbe einrasten, und daß die Kupplungsmuffe zumindestens im Bereich der Kerbe eine derartige Flexibilität besitzt, daß sie bei radialer Druckeinwirkung, senkrecht zu der die Vorsprünge verbindenden Linie, durch ihre Aufweitung von den Vorsprüngen freigegeben werden kann.

Der Vorteil der Erfindung besteht im wesentlichen darin, daß durch die über das Rastglied geschobene Kupplungsmuffe eine radiale Aufweitung des Schlauches an dieser Stelle verhindert wird und das Rastglied in der Hinterschneidung verbleibt. Für das Rastglied hat die Kupplungsmuffe die Funktion einer festen Einschellung. Die Kupplungsmuffe wird auf dem Schlauch in der Weise fixiert, daß die Vorsprünge des Rohrstutzens in die gegenüberliegende Kerbe der Kupplungsmuffe einrasten. Zum schnellen Lösen der Schlauchverbindung wird die Kupplungsmuffe durch seitliche Druckeinwirkung ellipsenförmig aufgeweitet, bis die Vorsprünge aus der Kerbe ausrasten und die Kupplungsmuffe über den Schlauch vom Rohrstutzen weggeschoben werden kann. Die Vorsprünge am Rohrstutzen sind gegenüberliegend, um 180 Grad versetzt, angeordnet und die Druckeinwirkungsrichtung erfolgt senkrecht zu der die Vorsprünge verbindenden Linie. Das Rastglied an der Innenseite des Schlauches kann als umlaufender Wulst oder in Form von einzelnen, wulstartigen Abschnitten ausgeführt sein.

Vorteilhafte Ausgestaltungen der Erfindung sind in den Unteransprüchen angegeben.

Es ist vorteilhaft, zur Kodierung der Kupplungseinrichtung Rohrstutzen vorzusehen mit Stutzenanschlüssen der Längen L1 und L2 und die Schläuche an den Schlauchenden mit korrespondierenden Schlauchanschlüssen der Längen L1 und L2 auszuführen. Eine Kodierung der Kupplungseinrichtung kann notwendig sein, um ein Vertauschen von Schläuchen zu verhindern, z.B. Inspirations- und Exspirationsschlauch in einem Atemschutzgerät.

Als Montagehilfe für das Aufschieben der Schläuche auf die Rohrstutzen ist es zweckmäßig, die Stutzenanschlüsse und die Schlauchanschlüsse konisch auszubilden. Beim Aufschieben des Schlauches wird das Rastglied durch die Konizität des Stutzenanschlusses allmählich aufgeweitet und schnappt dann in die Hinterschneidung des Rohrstutzens.

Ein Ausführungsbeispiel der Erfindung ist in der Zeichnung dargestellt und im folgenden näher erläutert.

Es zeigen:
- Fig. 1: eine Kupplungseinrichtung in teilweise aufgeschnittener Seitenansicht,
- Fig. 2: die Kupplungsmuffe in Seitenansicht im Schnitt,
- Fig. 3: die Aufsicht auf den Rohrstutzen,
- Fig. 4: die Seitenansicht einer kodierten Kupplungseinrichtung im Schnitt.

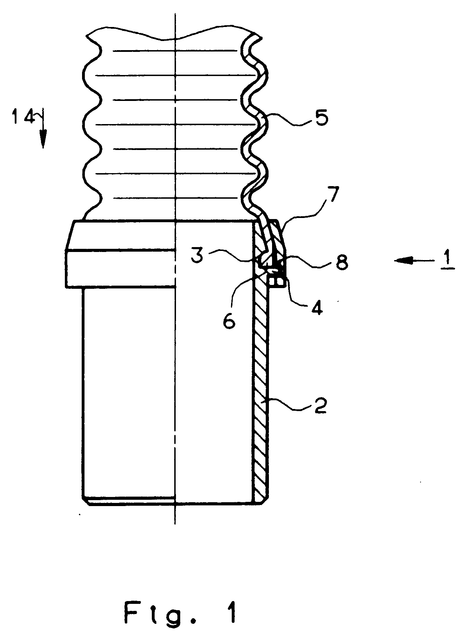

Die in der Fig. 1 dargestellten Kupplungseinrichtung (1) besteht aus einem Rohrstutzen (2) mit zwei Vorsprüngen (4), von denen nur einer in der Fig. 1 dargestellt ist und einem Schlauch (5) mit einem Rastglied (6), welches von einer Hinterschneidung (3) des Rohrstutzens (2) aufgenommen ist. Im Bereich des Rastgliedes (6) wird der Schlauch (5) schellenförmig durch eine Kupplungsmuffe (7) gesichert, die an dem Schlauch (5) anliegt. Die Kupplungsmuffe (7) ist an der Innenseite mit einer umlaufenden Kerbe (8) versehen, in die die Vorsprünge (4) einrasten, wodurch die Lage der Kupplungsmuffe (7) auf dem Schlauch (5) fixiert ist.

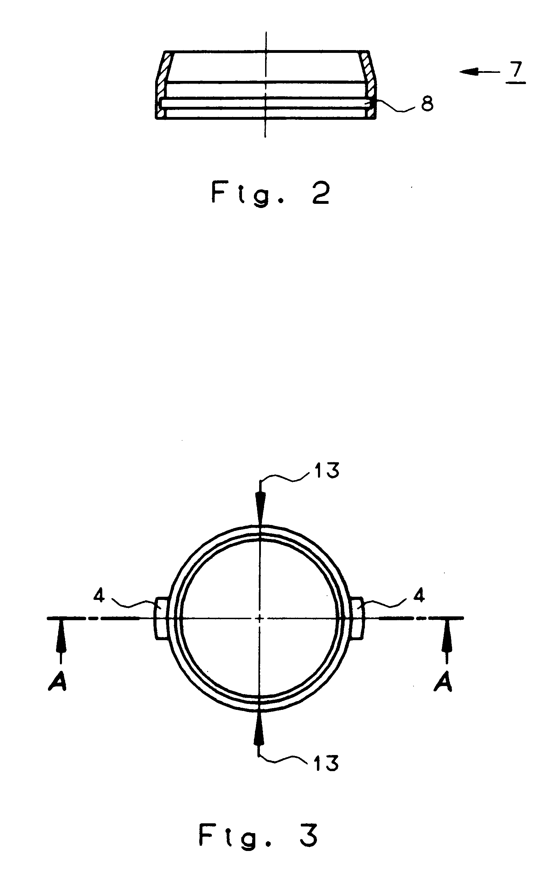

Fig. 2 zeigt eine Seitenansicht der Kupplungsmuffe (7) im Schnitt. An der Innenseite ist die umlaufende Kerbe (8) aus der Fig. 1 erkennbar.

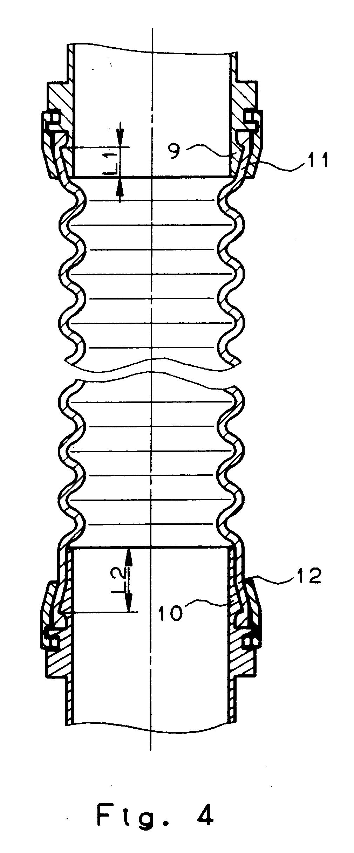

Fig. 3 veranschaulicht eine Aufsicht auf den Rohrstutzen (2), Fig. 1, mit den zwei gegenüberliegenden Vorsprüngen (4). Der rechte Teil der Schnittdarstellung A-A in der Fig. 3 entspricht dem rechten, aufgeschnittenen Teil der Fig. 1.

Die Kupplungseinrichtung (1), Fig. 1, wird, wie im folgenden beschrieben, zusammengesteckt.

Zunächst wird der Schlauch (5) über den vorderen Teil des Rohrstutzens (2) geschoben, bis das Rastglied (6) in der Hinterschneidung (3) einrastet, Fig. 1. Anschließend wird die Kupplungsmuffe (7) längs des Schubpfeils (14) über den Schlauch (5) geschoben, bis die Vorsprünge (4) in die Kerbe (8) einrasten.

Zum Lösen der Kupplungseinrichtung (1) wird die Kupplungsmuffe (7) in Richtung der Pfeile 13, Fig. 3, Fig. 1, zusammengedrückt, wodurch sich deren Querschnitt elliptisch verformt und die beiden Vorsprünge (4) aus der Kerbe (8) herausspringen. Die Kupplungsmuffe (7) kann nun in entgegengesetzter Richtung des Schubpfeils (14) vom Schlauch (5) geschoben werden. Danach wird der Schlauch (5) vom Rohrstutzen (2) abgezogen, wobei das Rastglied (6) aus der Hinterschneidung (3) herausspringt.

Fig. 4 zeigt die Seitenansicht einer kodierten Kupplungseinrichtung im Schnitt. Gegenüber der in der Fig. 1 dargestellten Kupplungseinrichtung (1) ist unterschiedlich, daß die Rohrstutzen (2) mit einem ersten Stutzenanschluß (9) der Länge L1 und einem zweiten Stutzenanschluß (10) der Länge L2 versehen sind, auf die jeweils ein erster Schlauchanschluß (11) der Länge L1 und ein zweiter Schlauchanschluß (12) der Länge L2 aufgeschoben ist. Die Kodierung wird im vorliegenden Fall dadurch erreicht, daß der zweite Schlauchanschluß (12) zwar auch auf den ersten Stutzenanschluß (9) aufgeschoben werden kann, im umgekehrten Fall aber der erste Schlauchanschluß (11) nicht auf den zweiten Stutzenanschluß (10) paßt. Die Stutzenanschlüsse (9, 10) und die Schlauchanschlüsse (11, 12) sind im Überlappungsbereich korrespondierend konisch ausgebildet, um die Montage des Schlauches (5) zu vereinfachen. Beim Aufschieben der Schlauchanschlüsse (11, 12) auf die zugehörigen Stutzenanschlüsse (9, 10) wird nämlich das Rastglied (6), Fig. 1, durch die Konizität der Stutzenanschlüße (9, 10) allmählich aufgeweitet, bis es in die Hinterschneidung (3), Fig. 1, einschnappt.

## Patentansprüche

1. Kupplungseinrichtung zwischen zwei Teilen eines Schlauchsystems, von denen das erste Teil ein Rohrstutzen (2) mit einer Hinterschneidung und das zweite Teil ein auf den Rohrstutzen (2) geschobener Schlauch mit einem in die Hinterschneidung (3) eingreifenden Rastglied (6) ist, dadurch gekennzeichnet, daß eine hülsenförmige Kupplungsmuffe (7) vorgesehen ist, die den Schlauch (5) im Bereich des Rastgliedes (6) als Sicherungsring schellenförmig umgreift und zur Fixierung auf dem Rohrstutzen (2) an der Innenseite eine umlaufende Kerbe (8) aufweist, daß am Rohrstutzen (2) zwei gegenüberliegend angeordnete Vorsprünge (4) vorgesehen sind, welche in die Kerbe (8) einrasten, und daß die Kupplungsmuffe (7) zumindestens im Bereich der Kerbe (8) eine derartige Flexibilität besitzt, daß sie bei radialer Druckeinwirkung, senkrecht zu der die Vorsprünge (4) verbindenden Linie, durch ihre Aufweitung von den Vorsprüngen freigegeben werden kann.

2. Kupplungseinrichtung nach Anspruch 1, dadurch gekennzeichnet, daß zur Kodierung Rohrstutzen mit einem ersten Stutzenanschluß (9) der Länge L1 und einem zweiten Stutzenanschluß (10) der Länge L2 vorgesehen sind und die Schläuche an den Enden jeweils einen ersten Schlauchanschluß (11) der Länge L1 und einen zweiten Schlauchanschluß (12) der Länge L2 besitzen.

3. Kupplungseinrichtung nach Anspruch 2, dadurch gekennzeichnet, daß die Stutzenanschlüsse (9, 10), die Schlauchanschlüsse (11, 12) und die Kupplungsmuffe (7) im Überlappungsbereich korrespondierend konisch ausgebildet sind.

## Claims

1. Coupling device between two parts of a tube system, the first part of which is a pipe socket (2) with an undercut and the second part is a tube pushed on to the pipe socket (2) and having a catch element (6) engaging into the undercut (3), characterized in that a sleeve-shaped coupling joint (7) is provided which engages in a clip-like manner around the tube (5) in the region of the catch element (6) as a securing ring and has at the inner side a circumferential indentation (8) for fixing to the pipe socket (2), in that two projections (4) arranged opposite each other are provided on the pipe socket (2), the projections latching into the indentation (8), and in that the coupling joint (7) has at least in the region of the indentation (8) such a flexibility that upon radial pressure action, perpendicular to the line connecting the projections (4), it can be released from the projections through its widening.

2. Coupling device according to claim 1, characterized in that pipe sockets with a first socket attachment (9) of the length L1 and a second socket attachment (10) of the length L2 are provided for coding and the tubes at the ends in each case have a first tube attachment (11) of the length L1 and a second tube attachment (12) of the length L2.

3. Coupling device according to claim 2, characterized in that the socket attachments (9, 10), the tube attachments (11, 12) and the coupling joint (7) are formed correspondingly conically in the overlapping region.

## Revendications

1. Dispositif d'accouplement entre deux parties d'un ensemble de tuyaux, dont la première partie est une tubulure (2) pourvue d'une contre-dépouille et la deuxième partie est un tuyau enfoncé sur la tubulure (2), pourvu d'un organe d'enclenchement (6) s'engageant dans la contre-dépouille (3), caractérisé en ce qu'est prévu un manchon d'accouplement (7) en forme de douille, qui entoure le tuyau (5), en le bridant, dans la zone de l'organe d'enclenchement (6), en agissant comme un circlip et qui présente, sur la face intérieure, pour la fixation sur la tubulure (2), une encoche (8) circonférentielle, en ce que deux saillies (4) opposées sont prévues sur la tubulure (2) et s'enclenchent dans l'encoche (8), et en ce que le manchon d'accouplement (7), au moins dans la zone de l'encoche (8), présente une flexibilité telle qu'il puisse être libéré, par son élargissement vis-à-vis des saillies, lorsqu'est appliquée une pression radiale perpendiculairement à la ligne réunissant les saillies (4).

2. Dispositif d'accouplement selon la revendication 1, caractérisé en ce que sont prévues, pour le codage, des tubulures pourvues d'un premier raccord de tubulure (9) de longueur L1 et d'un deuxième raccord de tubulure (10) de longueur L2, et en ce que les tuyaux présentent des extrémités pourvues, chacune, d'un premier raccord de tuyau (11) de longueur L1 et d'un deuxième raccord de tuyau (12) de longueur L2.

3. Dispositif d'accouplement selon la revendication 2, caractérisé en ce que les raccords de tubulures (9, 10), les raccords de tuyaux (11, 12) et le manchon d'accouplement (7) ont une forme conique correspondante, dans la zone de recouvrement.
